(19) Europäisches Patentamt
European Patent Office
Office européen des brevets

(11) Veröffentlichungsnummer: **0 475 255 A2**

(12) **EUROPÄISCHE PATENTANMELDUNG**

(21) Anmeldenummer: **91114879.9**

(51) Int. Cl.[5]: **C12P 41/00**, C12P 9/00

(22) Anmeldetag: **04.09.91**

(30) Priorität: **12.09.90 CH 2956/90**

(43) Veröffentlichungstag der Anmeldung: **18.03.92 Patentblatt 92/12**

(84) Benannte Vertragsstaaten: **AT BE CH DE DK FR GB IT LI NL**

(71) Anmelder: **F. HOFFMANN-LA ROCHE AG**
**Postfach 3255**
**CH-4002 Basel(CH)**

(72) Erfinder: **Wirz, Beat**
**Wiedenweg 4**
**CH-4153 Reinach(CH)**
Erfinder: **Wostl, Wolfgang**
**Im Strick 2**
**W-7889 Grenzach-Wyhlen(DE)**

(74) Vertreter: **Kellenberger, Marcus, Dr. et al**
**Grenzacherstrasse 124 Postfach 3255**
**CH-4002 Basel(CH)**

# (54) Verfahren zur Herstellung optisch reiner (S)-alpha-[(tert-Butylsulfonyl)methyl]hydrozimtsäure.

(57) Optisch reine (S)-α-[(tert-Butylsulfonyl)methyl]hydrozimtsäure der Formel

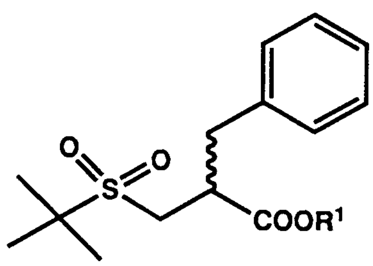

II

wird durch Protease-katalysierte selektive Hydrolysde in Emulsion in einem wässrigen Medium bei einem kontrollierten pH-Wert zwischen etwa 6,5 und etwa 8,5 aus racemischen (RS)-α-[(tert-Butylsulfonyl)methyl]-hydrozimtsäureestern der Formel

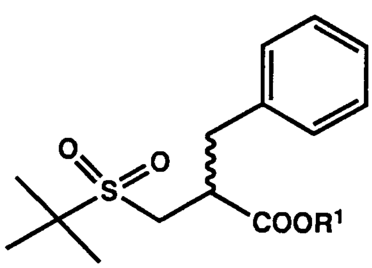

II

worin $R^1$ $C_{1-4}$-Alkyl bedeutet,
hergestellt.

EP 0 475 255 A2

Die vorliegende Erfindung betrifft ein neues Verfahren zur Herstellung optisch reiner (S)-α-[(tert-Butylsulfonyl)methyl]hydrozimtsäure der Formel

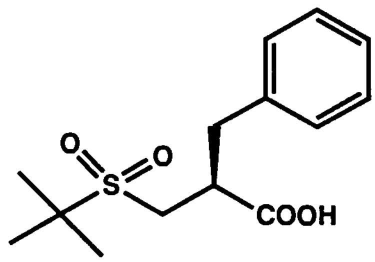

I

durch enzymatisch-kinetische Aufspaltung von racemischen (RS)-α-[(tert-Butylsulfonyl)methyl]-hydrozimtsäureestern der allgemeinen Formel

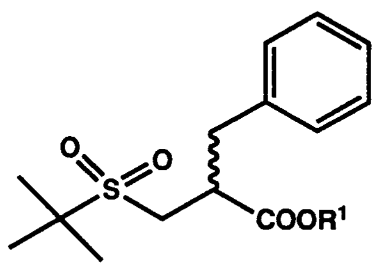

II

worin $R^1$ $C_{1-4}$-Alkyl bedeutet.

Der in dieser Beschreibung verwendete Ausdruck "$C_{1-4}$-Alkyl" betrifft geradkettige und verzweigte Alkylgruppen mit 1-4 Kohlenstoffatomen, wie Methyl, Aethyl, Propyl, Isopropyl, Butyl und dergleichen, vorzugsweise Methyl oder Aethyl, besonders bevorzugt Aethyl.

In den in der vorliegenden Beschreibung angegebenen Formeln bedeutet eine dicke, spitzzulaufende Linie (▲) einen Substituenten mit β-Orientierung (d.h. oberhalb der Ebene des Moleküls bzw. der Seite), eine gestrichelte Linie ( ≐) einen Substituenten mit α-Orientierung (d.h. unterhalb der Ebene des Moleküls bzw. der Seite) und eine Wellenlinie ( ⌇) einen Substituenten mit α- oder β-Orientierung oder Gemische dieser Isomeren.

Erfindungsgemäss wurde gefunden, dass das S-Enantiomere der Formel II selektiv zum S-Enantiomeren der Formel I hydrolysiert wird, wenn man das racemische Gemisch der Formel II einer enzymatischen Hydrolyse unter Verwendung eines Protease-Enzyms unterwirft.

Die erfindungsgemässe enzymatische Hydrolyse fuhrt demnach zum 2R-Enantiomeren einer Verbindung der Formel II, d.h. einer Verbindung der allgemeinen Formel

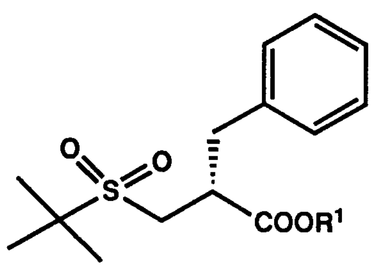

IIA

worin $R^1$ die oben angegebene Bedeutung besitzt,
im Gemisch mit dem 2S-Enantiomeren der Formel I. Diese Verbindungen können danach leicht nach üblichen Methoden getrennt werden.

Die Selektivität gewisser Mikroorganismen oder gewisser Enzyme, welche aus Mikroorganismen oder Vertebratenorganen gewonnen wurden, ermöglicht deren potentielle Verwendung zur Herstellung von enantiomer reinen Zwischenprodukten aus racemischen Gemischen. Das erwünschte enantiomere Molekül kann danach in die Zielverbindung übergeführt werden. Die durch einen Mikroorganismus oder ein Enzym

katalysierte Aufspaltung von Isomeren ergibt eine attraktive Alternative zu den mehr traditionellen und kostspieligen Methoden, wie chemische Aufspaltung und Hochdruck-Flüssigchromatographie diastereomerer Derivate.

Kato et al. haben berichtet, dass Corynebacterium equi IFO 3730 die Eigenschaft besitzt, verschiedene Ester enantioselektiv zu hydrolysieren (Tetrahedron Letters, Band 28, Nr. 12,1987, Seiten 1303-1306). In ihrer Studie wurde der Mikroorganismus für die asymmetrische Hydrolyse 2-Benzyloxy-substituierter Alkan- und Arylalkancarbonsäureester eingesetzt. Die nicht reagierten Niederalkylester wurden in der optisch aktiven S-Form wiedergewonnen, und zwar in einem hohen enantiomeren Ueberschuss (>99% e.e.). Es wurde auch festgestellt, dass der Austausch des Alkyl- oder Alkenylrestes des Substrates mit einer Phenylmethylgruppe eine Umkehrung der Stereoselektivität verursachte, d.h. die Verbindung fiel in der optisch aktiven R-Form an, und zwar ebenfalls in hoher enantiomerer Reinheit.

Kitazume et al. haben ein Verfahren zur asymmetrischen Hydrolyse von 2-Fluor-2-methylmalonsäurediestern mit Esterase aus Schweineleber beschrieben, gemäss welchem die optisch aktiven (-)-2-Fluor-2-methylmalonsäuremonoester erhalten wurden, jedoch in niedriger enantiomerer Reinheit. Beschrieben wurde auch eine mikrobielle Hydrolyse von 2-Fluor-2-substituierten Malonsäurediestern mit Esterase und Cellulase, wobei die optisch aktiven (+)- oder (-)-2-Fluor-2-substituiertenMalonsäuremonoester erhalten wurden (J. Org. Chem., 51, 1986, Seiten 1003-1006).

Iuchijima et al. haben ein Verfahren zur Herstellung optisch aktiver 2-Chlor- und 2-Brom-substituierter Alkylester und -säuren durch asymmetrische Hydrolyse racemischer Gemische der Ester beschrieben, wobei die Mikroorganismen Rhizopus, Mucor, Aspergillus, Candida, Pseudomonas, Alcaligenes, Achromobacter und Bacillus, oder aus diesen Mikroorganismen gewonnene Enzyme verwendet wurden (publizierte Japanische Patentanmeldung [Kokai] Nr. 57-94.295 [1982]).

U.S. Patentschrift Nr. 4,668,628 (Dahod et al.) beschreibt ein Verfahren zur enzymatischen Spaltung racemischer Gemische von partiell wasserlöslichen Estern, welches darin besteht, dass man das racemische Gemisch mit einer Candida Lipase in Kontakt bringt, um dieses enzymatisch zu hydrolysieren. Ein spezifisches Beispiel ist die Candida Lipase-katalysierte Hydrolyse von D,L-2-Chlorpropionsäuremethylester.

Die Europäischen Patentpublikationen 0.325.954 und 0.325.971 (beide Coffen et al.) beschreiben ein Verfahren zur enzymatischen Spaltung racemischer, Hydroxy-substituierter Benzopyrancarbonsäureester bzw. Arylalkansäureester, welches darin besteht, dass man das racemische Gemisch mit einer Pseudomonas Lipase in Lösung oder in Suspension in einem wässrigen Medium bei einem kontrollierten pH-Wert zwischen ungefähr 5 und ungefähr 10 bzw. 9 umsetzt, um dieses enzymatisch zu hydrolysieren.

Björkling et al. haben ein Verfahren beschrieben, gemäss welchem prochirale oder racemische Arylmethyl-substituierte Malonsäuredialkylester durch $\alpha$-Chymotrypsin-katalysierte enantioselektive Hydrolyse in die entsprechenden optisch aktiven Monoalkylester übergeführt werden können (Biocatalysis, 1987, Band 1, Seiten 87-98).

Pugnière et al. haben berichtet, dass optisch aktive aromatische Aminosäuren durch Protease-katalysierte enantioselektive Hydrolyse ihrer entsprechenden Alkylester in Form von Emulsionen von Wasser mit organischen, mit Wasser nicht mischbaren Lösungsmitteln hergestellt werden können, wobei die Protease auf Aluminiumoxyd fixiert ist (Biotechnology Techniques, Band 3, Nr. 5,1989, Seiten 339-344).

Ein gewichtiger Nachteil Enzym-katalysierter kinetischer Spaltungen ist insbesondere die Tatsache, dass die Spezifität des Enzyms für ein gegebenes Substrat meist nicht vorausgesagt werden kann, da keine nützlichen Modelle existieren, welche für eine Enzym-katalysierte kinetische Aufspaltung eines potentiellen Substrates eine Voraussage bezüglich der Stereochemie ermöglichen.

Zur Durchführung der erfindungsgemässen enzymatischen Aufspaltung wird die Verbindung der Formel II zunächst unter Erwärmen, gegebenenfalls in Gegenwart eines Cosolvens, mit einer wässrigen Phase emulgiert, und diese anschliessend mit dem Enzym versetzt. Das Emulgieren erfolgt bei einer Temperatur von 15-55 °C, vorzugsweise 35-45 °C, so dass die Verbindungen der Formel II teilweise in Form einer Schmelze emulgiert werden. Die Emulsion enthält 0,1-15% (G/V), vorzugsweise 6-10%, der Verbindung der Formel II. Wird ein Cosolvens verwendet, so beträgt dessen Anteil 0,1-5% (V/V), vorzugsweise 1,0-1,5%. Als Cosolventien kommen die üblichen, mit Wasser mischbaren, polaren organischen Lösungsmittel, wie Alkohole, z.B. Methanol oder Aethanol, Aceton, Dimethylsulfoxid und dgl. in Frage. Als wässrige Phase dient Wasser, wobei vorzugsweise gewöhnliches Hahnenwasser verwendet wird, welches erwünschtenfalls noch Salze, wie Calciumchlorid, Magnesiumchlorid, Natriumchlorid und dgl., oder Puffersalze, wie Natriumphosphat und dgl. enthalten kann. Die Menge an Salz wird vorzugsweise so gewählt, dass die wässrige Phase 5-500 mM ist.

Als Proteasen-Enzyme können solche verwendet werden, die aus Vertebraten oder Mikroorganismen, vorzugsweise Mikroorganismen, gewonnen werden, wie a-Chymotrypsin, HT Proteolytic 200 (Miles Kali-Chemie GmbH & Co, 3000 Hannover-Kleefeld), Optimase (Miles Kali-Chemie GmbH & Co, 3000 Hannover-Kleefeld), Savinase (NOVO, 2880 Bagsvaerd, Dänemark), Alcalase (NOVO, 2880 Bagsvaerd, Dänemark), Prozyme (Amano Pharmaceutical Co, Ltd, Nagoya, Japan) und dgl. Besonders bevorzugt ist die Verwendung von Optimase, Savinase und Alcalase, ganz besonders bevorzugt die von Optimase. Das Mengenverhältnis von Verbindung der Formel II zu Enzym wird so gewählt, dass die Hydrolyse nach etwa 24 Stunden beendet ist.

Die enzymatische Hydrolyse wird bei einem pH von ungefähr 6,5 bis 8,5, vorzugsweise bei einem pH von ungefähr 7,5, durchgeführt. Um den pH-Wert des Reaktionsgemisches im vorgenannten Bereich halten zu können, kann jede übliche Methode verwendet werden. Unter den bevorzugten Methoden kann die automatische Titration mit einer Base oder die Verwendung von Puffern genannt werden. Die automatische Titration wird zweckmässigerweise mit einer wässrigen Base, wie Natron- oder Kalilauge, durchgeführt. Dabei wird vorteilhafterweise eine 0,1-5M, vorzugsweise 1-2M, Lösung eingesetzt.

Bei der Durchführung dieser enzymatischen Hydrolyse wird das racemische Gemisch der Formel II in einem wässrigen Medium emulgiert und mit vorzugsweise bakterieller Protease umgesetzt. Im allgemeinen ist es bevorzugt, das Enzym in einer katalytisch wirksamen Menge zu verwenden. Unbestrittenermassen wird die zum Erhalten bester Resultate notwendige Bestimmung einer katalytisch wirksamen Menge eines bestimmten Enzyms von Faktoren abhängen, welche einem Fachmann geläufig sind. Diese Faktoren umfassen die Menge Ausgangsmaterial, die Herkunft des Enzyms, die Aktivität des Enzyms, die Reinheit des Enzyms und dergleichen. Man kann zwar einen Ueberschuss einer katalytisch wirksamen Menge der Protease verwenden, doch wird durch die Verwendung eines grossen Ueberschusses an Enzym keine Verbesserung des Resultats erreicht.

Wie oben erwähnt, liefert die enzymatische Hydrolyse der racemischen Verbindung der Formel II die Verbindung der Formel I im Gemisch mit der Verbindung der Formel IIA. Diese Verbindungen können leicht getrennt werden, wenn die enzymatische Hydrolyse einmal gestoppt ist, und zwar durch sofortige Extraktion des Reaktionsmediums mit einem geeigneten organischen Lösungsmittel. Jede übliche Methode zur Trennung kann verwendet werden, um die Verbindung der Formel I von der Verbindung der Formel IIA zu isolieren. Unter den üblichen Methoden zur Trennung dieser zwei Verbindungen können Extraktion und Chromatographie genannt werden.

Ein wichtiger Aspekt des erfindungsgemässen Verfahrens ist das Erreichen einer einfachen, effizienten und ökonomischen Racematspaltung durch die Kombination von geeigneten speziellen Reaktionsbedingungen, wie das Emulgieren des Substrates durch Wärme, die Verwendung einfacher wässriger Medien, der Einsatz einer hohen Substratkonzentration und die Verwendung einer Protease, insbesondere einer billigen mikrobiellen Protease, die unter diesen speziellen Bedingungen (erhöhte Temperatur und hohe Substratkonzentration) nicht nur aktiv, sondern auch enantioselektiv bleibt.

Die nach dem erfindungsgemässen Verfahren erhältlichen Verbindungen sind nützliche Zwischenprodukte zur Herstellung von Renin-Hemmern, die zur Behandlung von Bluthochdruck verwendet werden können. Verfahren zur Herstellung solcher Renin-Hemmer aus Verbindungen wie den erfindungsgemäss Herstellbaren sind dem Fachmann geläufig und sind in der Patentliteratur beschrieben, beispielsweise in EPA 0 332 008 (Branca et al.).

Die folgenden Beispiele illustrieren im weiteren die vorliegende Erfindung, ohne diese zu beschränken.

Zur Bestimmung der enantiomeren Reinheit wurde die erhaltene α-[(tert-Butylsulfonyl)methyl]-hydrozimtsäure mit (R)-(+)-α-Methyl-p-nitrobenzylamin nach einer Methode von E. Bald, K. Saigo und T. Mukaijama (Chem. Letters, 1975, 1163) umgesetzt. Die erhaltenen Diastereomeren wurden gaschromatographisch über eine Kapillarsäule PS 086(15 m, 50-300°C) aufgetrennt.

## Beispiel 1

1,60 g (5,12 mMol) (RS)-α-[(tert-Butylsulfonyl)methyl]hydrozimtsäureäthylester wurden mit 50 ml wässriger 5 mM Calciumchloridlösung versetzt, und das Reaktionsgemisch unter Rühren auf 38°C erwärmt. Der pH-Wert wurde mit 1N Natronlauge auf 7,5 eingestellt. Durch Zugabe von 150 mg Savinase 6,0 T (NOVO, 2880 Bagsvaerd, Dänemark) wurde die Hydrolyse gestartet. Der pH-Wert wurde mit automatischer Titration durch Zudosieren von 1N Natronlauge unter starkem Rühren bei 38°C bei 7,5 konstant gehalten. Nach einem Verbrauch von 2,39 ml 1N Natronlauge (nach 21,3 Stunden) wurde das Reaktionsgemisch mit zweimal 50 ml Aethylacetat gewaschen. Die wässrige Phase wurde mit 25%iger Salzsäure auf pH 2,2 angesäuert und mit zweimal 50 ml Aethylacetat extrahiert. Die organische Phase wurde über Magnesiumsulfat getrocknet und bei 1200 Pa/40°C eingedampft, wobei man 690 mg (2,42 mMol, 47%, 94% der Theorie) (S)-α-[(tert-Butylsulfonyl)methyl]hydrozimtsäure in Form weisser Kristalle erhielt, welche einen enantiomeren Ueberschuss von >99% aufwies. Zur Bestimmung der Reinheit im Gaschromatographen

wurde die erhaltene Verbindung silyliert und wies ebenfalls eine Reinheit von >99% auf.

Beispiel 2

In zu Beispiel 1 analoger Weise wurde die Hydrolyse von 1,60 g (5,12 mMol) (RS)-α-[(tert-Butylsulfonyl)methyl]hydrozimtsäureäthylester in Gegenwart von 25 ml wässriger 5 mM Calciumchloridlösung und 100 mg Alcalase 2,0 T (NOVO, 2880 Bagsvaerd, Dänemark) durchgeführt. Nach einem Verbrauch von 2,47 ml 1N Natronlauge (nach 21,3 Stunden) wurde das Reaktionsgemisch analog Beispiel 1 aufgearbeitet, wobei 700 mg (2,46 mMol, 48%, 96% der Theorie) (S)-α-[(tert-Butylsulfonyl)methyl]hydrozimtsäure erhalten wurden, welche sowohl einen enantiomeren Ueberschuss als auch eine GC-Reinheit von je >99% aufwies.

Beispiel 3

Zu 97 l Hahnenwasser wurden 54 g Calciumchlorid gegeben, sodass eine 5 mM Lösung entstand. Die Lösung wurde unter Rühren auf 39-40° C thermostatisiert. 6,50 kg geschmolzener (RS)-α-[(tert-Butylsulfonyl)methyl]hydrozimtsäureäthylester (87% GC) wurden unter Rühren langsam zur warmen Lösung gegossen. Der pH-Wert der Emulsion betrug 7,9. Die Hydrolyse wurde durch Zugabe von 300 g Optimase M 440 gestartet und der pH-Wert durch Zudosieren von 1N Natronlauge mittels automatischer Titration unter Rühren bei 7,5 konstant gehalten. Nach einem Verbrauch von 9,6 l 1N Natronlauge (nach 22,5 Stunden) wurden unter Rühren zum Reaktionsgemisch 3 kg DICALITE Speedex und 10 l Aethylacetat gegeben, und die Suspension filtriert. Das Filtrat wurde anschliessend mit 40,30 und 20 l Aethylacetat (Totalmenge 90 l) extrahiert. Zur schnelleren Phasentrennung wurden 2, 0,5 bzw. 0,2 kg Natriumsulfat (Totalmenge 2,7 kg) jeweils zugegeben. Die drei organischen Phasen wurden über Magnesiumsulfat getrocknet und bei 1200 Pa/40° C eingedampft, wobei 2,39 kg (R)-α-[(tert-Butylsulfonyl)methyl]-hydrozimtsäureäthylester erhalten wurden. Die wässrige Phase wurde mit 1,5 l 25%iger Salzsäure auf pH 2,2 eingestellt und mit 25 und 20 l (Totalmenge 45 l) Aethylacetat extrahiert. Die erste Phasentrennung wurde durch Zugabe von 0,2 kg Natriumsulfat beschleunigt. Die beiden organischen Phasen wurden über Magnesiumsulfat getrocknet und bei 1200 Pa/40° C eingedampft, wobei 2,22 kg (7,716 Mol) (S)-α-[(tert-Butylsulfonyl)methyl]hydrozimtsäure, welche einen enantiomeren Ueberschuss und eine gaschromatographische Reinheit von je >99% aufwies, erhalten wurden.

Beispiel 4

Eine Lösung von 79 g (0,25 Mol) (RS)-α-[(tert-Butylsulfonyl)methyl]hydrozimtsäureäthylester in 105 ml Dimethylsulfoxid wurde unter starkem Rühren zu 6,2 l Wasser von 30° C getropft. Der pH-Wert wurde mit 0,1N Natronlauge auf 7,5 eingestellt. Durch Zugabe von 1,05 g α-Chymotrypsinwurde die Hydrolyse gestartet. Der pH-Wert wurde mit automatischer Titration durch Zudosieren von 0,043N Calciumhydroxidlösung bei 30° C bei 7,5 konstant gehalten. Nach einem Verbrauch von 2,67 l 0,043N Calciumhydroxidlösung (nach 19,5 Stunden) wurde das Reaktionsgemisch mit dreimal 2 l Aethylacetat extrahiert. Die wässrige Phase wurde mit 5%iger Salzsäure auf pH 2,5 angesäuert und mit zweimal 2 l Aethylacetat extrahiert. Die organischen Phasen wurden über DICALITE Speedex filtriert, vereinigt und über Magnesiumsulfat getrocknet. Nach dem Eindampfen des Lösungsmittels und Trocknen des Rückstandes am Hochvakuum erhielt man 35 g (0,12 Mol, 48,3%, 96,6% der Theorie) (S)-α-[(tert-Butylsulfonyl)methyl]hydrozimtsäure in Form weisser Kristalle, welche einen enantiomeren Ueberschuss von >98% aufwies. Zur Bestimmung der Reinheit im Gaschromatographen wurde die erhaltene Verbindung silyliert und wies eine Reinheit von >99% auf.

**Patentansprüche**

**1.** Verfahren zur Herstellung optisch reiner (S)-α-[(tert-Butylsulfonyl)methyl]hydrozimtsäure der Formel

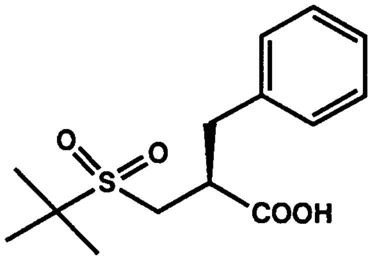

I

dadurch gekennzeichnet, dass man einen racemischen (RS)-α-[(tert-Butylsulfonyl)methyl]-hydrozimtsäureester der allgemeinen Formel

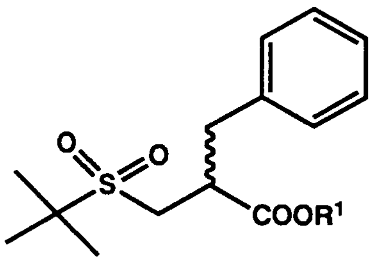

II

worin $R^1$ $C_{1-4}$-Alkyl bedeutet,
in einem wässrigen Medium emulgiert und mit einer Protease zur selektiven Ueberführung des racemischen (RS)-Esters in die (S)-Carbonsäure umsetzt, wobei die Umsetzung bei einem pH-Wert zwischen etwa 6,5 und etwa 8,5 durchgeführt wird, und danach den (R)-Ester und die (S)-Säure getrennt aus dem Reaktionsmedium isoliert.

**2.** Verfahren gemäss Anspruch 1, worin das Substrat durch Schmelzen bei erhöhter Temperatur emulgiert wird.

**3.** Verfahren gemäss Anspruch 1 oder 2, worin die Reaktion bei einem pH-Wert von etwa 7,5 durchgeführt wird.

**4.** Verfahren gemäss einem der Ansprüche 1-3, worin $R^1$ Aethyl bedeutet.

**5.** Verfahren gemäss einem der Ansprüche 1-4, worin das wässrige Medium Calciumchlorid enthält.

**6.** Verfahren gemäss einem der Ansprüche 1-5, worin die Protease aus einem Mikroorganismus gewonnen wurde.

**7.** Verfahren gemäss Anspruch 6, worin es sich bei der Protease um ein bakterielles Enzym handelt.

**8.** Verfahren gemäss Anspruch 7, worin es sich bei der Protease um Optimase, Savinase oder Alcalase handelt.